# EUROPÄISCHE PATENTSCHRIFT

(11) **EP 0 622 366 B1**
(45) Veröffentlichungstag und Bekanntmachung des Hinweises auf die Patenterteilung: **04.12.1996**
(21) Anmeldenummer: 94105778.8
(22) Anmeldetag: 14.04.1994
(51) Int. Cl.: C07D 401/04, A61K 31/435

(54) **Kondensierte Chinolyl-dihydropyridine, Verfahren zu ihrer Herstellung und ihre Verwendung in Arzneimitteln zur Behandlung von Herz-Kreislauferkrangen**
Condensed quinolyl-dihydropyridines, process for their preparation and their use in medicine for the treatment of heart-circulatory illnesses
Quinolyl-dihydropyridines condensées, procédé pour leur préparation et leur utilisation comme médicament pour le traitement de maladies circulatoires du coeur

(30) Priorität: 27.04.1993 DE 4313690
(43) Veröffentlichungstag der Anmeldung: 02.11.1994
(73) Patentinhaber: BAYER AG, 51368 Leverkusen (DE)
(72) Erfinder: Goldmann, Siegfried, Dr., D-42327 Wuppertal (DE); Stolefuss, Jürgen, Dipl.-Ing., D-42781 Haan (DE); Straub, Alexander, Dr., D-42113 Wuppertal (DE); Bechem, Martin, Dr., D-42111 Wuppertal (DE); Gross, Rainer, Prof. Dr., D-42115 Wuppertal (DE); Hebisch, Siegbert, Dr., D-26244 Bottrop (DE); Hütter, Jochim, Dr., D-42349 Wuppertal (DE); Rounding, Howard-Paul, Dr., D-42115 Wuppertal (DE)

(56) Entgegenhaltungen:
- EP-A- 0 157 324
- EP-A- 0 452 712

## Beschreibung

Die Erfindung betrifft neue kondensierte 4-Chinolyl-dihydropyridine, Verfahren zu ihrer Herstellung und ihre Verwendung in Arzneimitteln, insbesondere in Mitteln zur Behandlung von Herzkreislauferkrankungen.

Es ist bereits bekannt, daß 1,4-Dihydropyridine gefäßerweiternde Eigenschaften besitzen und als Coronarmittel und Antihypertensiva verwendet werden können. Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können.
Auch für einige 4-Chinolyl-dihydropyridine ist bereits ihre positiv inotrope Wirkung bekannt, vergl. EP-A-452 712, in der die Tetrahydrofuropyridine beschrieben werden. In EP-A-157 324 werden Tetrahydrothienopyridine mit gleicher Wirkung beschrieben. Die erfindungegemäßen Dihydropyridine unterscheiden sich von diesen durch das Vorhandensein einer -CH₂-Gruppe im ankondensierten Fünfring anstelle der -O- bzw. -S-Gruppe.

Bei Kenntnis des Standes der Technik war es nicht vorhersehbar, daß die erfindungsgemäßen Verbindungen eine kontraktionskraftverstärkende, am Herzmuskel positiv inotrope Wirkung mit weitgehend gefäßneutralem Verhalten besitzen.

Die vorliegende Anmeldung betrifft kondensierte 4-Chinolyl-dihydropyridine der allgemeinen Formel (I) 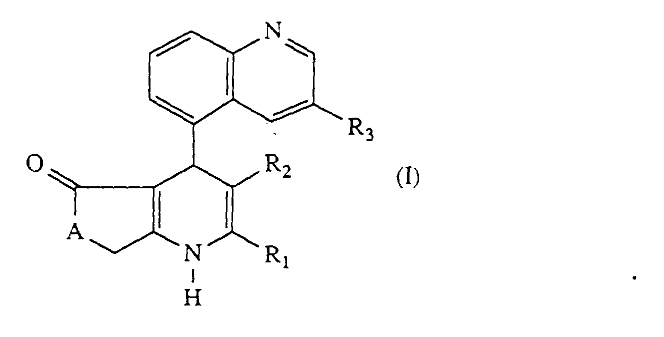 in welcher
- A: für die -CH₂-Gruppe steht,
- R¹: für Wasserstoff, Amino, Cyano, Formyl, Trifluormethyl oder für geradkettiges oder verzweigtes Alkyl mit bis zu 8 Kohlenstoffatomen steht, das gegebenenfalls durch Hydroxy oder durch eine Gruppe der Formel -NR⁶R⁷, -O-CO-R⁸, -O-(CH₂)ₐ-OR^{8'} oder -O-(CH₂)_{b'}-NR⁹R¹⁰ substituiert ist,
worin
R⁶, R⁷, R⁹ und R¹⁰ gleich oder verschieden sind und Wasserstoff, Phenyl oder geradkettiges oder verzweigtes Alkyl mit bis zu 6 Kohlenstoffatomen bedeuten,
R⁸ und R^{8'} gleich oder verschieden sind und geradkettiges oder verzweigtes Alkyl mit bis zu 6 Kohlenstoffatomen bedeuten, und
a und b gleich oder verschieden sind und eine Zahl 2, 3, 4 oder 5 bedeuten,
- R²: für eine Gruppe der Formel -CO-NR¹¹R¹² oder -CO-D-R¹³ steht,
worin
- R¹¹ und R¹²: gleich oder verschieden sind und Wasserstoff, einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
oder
- R¹¹ und R¹²: gemeinsam unter Einbezug des Stickstoffatoms einen 3- bis 8-gliedrigen, gesättigen oder ungesättigten Heterocyclus bilden, der gegebenenfalls durch ein Sauerstoffatom oder durch einen Rest der Formel S(O)_{d'} -CO- oder -NR¹⁵ unterbrochen ist,
worin
d eine Zahl 0, 1 oder 2 bedeutet,
R¹⁵ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl mit jeweils bis zu 8 Kohlenstoffatomen oder durch Halogenalkyl, Halogenalkoxy oder Halogenalkylthio mit jeweils bis zu 4 Kohlenstoffatomen substituiert ist, oder einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,
- D: eine direkte Bindung oder ein Sauerstoffatom bedeutet,
- R¹³: Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder
einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch -CO-, -CO-NH-, -O-CO-, -CO-O-, -NH-CO-, -SO₂-NH-, -NH-SO₂-, -S(O)ₑ-oder -NR¹⁶ unterbrochen ist,
worin
e die oben angegebene Bedeutung von d hat und mit dieser gleich oder verschieden ist,
R¹⁶ die oben angegebene Bedeutung von R¹⁵ hat und mit dieser gleich oder verschieden ist,
oder der Kohlenstoffrest gegebenenfalls bis zu 3-fach gleich oder verschieden durch Aryliden mit 6 bis 10 Kohlenstoffatomen oder heterocyclische Reste der Formeln 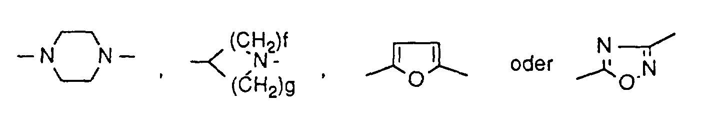 unterbrochen ist,
worin
f und g gleich oder verschieden sind und eine Zahl 1 oder 2 bedeuten,
und wobei die Aryl- und Hetero-Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,
und der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, -O-NO₂, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
der Kohlenwasserstoffrest durch eine Gruppe der Formel -CO₂-R¹⁷, -CONR¹⁸R¹⁹ oder -NR²⁰R²¹ substituiert sein kann,
worin
- R¹⁷: die oben angegebene Bedeutung von R¹⁵ hat und mit dieser gleich oder verschieden ist
und
- R¹⁸, R¹⁹, R²⁰ und R²¹: die oben angegebene Bedeutung von R¹¹ und R¹² haben und mit diesen gleich oder verschieden sind,
und
- R³: für Aryl mit 6 bis 10 Kohlenstoffatomen steht, das gegebenenfalls bis zu 2-fach gleich oder verschieden durch Halogen, Nitro, Cyano, Trifluormethyl, Trifluormethoxy, Trifluormethylthio, geradkettlges oder verzweigtes Alkyl, Alkoxy oder Alkoxycarbonyl mit jeweils bis zu 8 Kohlenstoffatomen oder Carboxy substituiert ist, oder für Thienyl oder Pyridyl steht, die gegebenenfalls durch Halogen substituiert sind
und deren Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher
- A: für die CH₂-Gruppe steht,
- R¹: für Wasserstoff, Amino, Cyano, Formyl, Trifluormethyl oder für geradkettiges oder verzweigtes Alkyl mit bis zu 6 Kohlenstoffatomen steht, das gegebenenfalls durch Hydroxy oder durch eine Gruppe der Formel -NR⁶R⁷, -O-CO-R⁸, -O-(CH₂)ₐ-OR^{8'} oder -O-(CH₂)_{b}, -NR⁹R¹⁰ substituiert ist,
worin
R⁶, R⁷, R⁹ und R¹⁰ gleich oder verschieden sind und Wasserstoff oder geradkettiges oder verzweigtes Alkyl mit bis zu 4 Kohlenstoffatomen bedeuten,
R⁸ und R^{8'} gleich oder verschieden sind und geradkettiges oder verzweigtes Alkyl mit bis zu 4 Kohlenstoffatomen bedeuten, und
a und b gleich oder verschieden sind und eine Zahl 2, 3 oder 4 bedeuten,
- R²: für eine Gruppe der Formel -CO-NR¹¹R¹² oder -CO-D-R¹³ steht,
worin
R¹¹ und R¹² gleich oder verschieden sind und Wasserstoff, einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor, Hydroxy, Cyano oder durch Phenyl, Phenyloxy, Phenylthio oder Pyridyl substituiert ist, wobei die Cyclen ihrerseits durch Fluor, Chlor oder durch Alkyl, Alkoxy, Alkoxycarbonyl mit jeweils bis zu 2 Kohlenstoffatomen, Trifluormethyl oder Trifluormethoxy substituiert sein können, oder Phenyl oder Pyridyl bedeuten, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind,
oder
- R¹¹ und R¹²: gemeinsam unter Einbezug des Stickstoffatoms einen 5- bis 7-gliedrigen, gesättigen oder ungesättigten Heterocyclus bilden, der gegebenenfalls durch ein Sauerstoffatom oder durch einen Rest der Formel S(O)_{d}, -CO- oder -NR⁵ unterbrochen ist,
worin
d eine Zahl 0, 1 oder 2 bedeutet,
R¹⁵ Wasserstoff oder Phenyl bedeutet, das gegebenenfalls durch Fluor, Chlor, durch geradkettiges oder verzweigtes Alkyl oder Alkoxy mit jeweils bis zu 4 Kohlenstoffatomen, Trifluormethyl oder Trifluormethoxy substituiert ist, oder einen cyclischen, gesättigten oder ungesättigten, geradkettigen oder verzweigten Kohlenwasserstoffrest mit bis zu 4 Kohlenstoffatomen bedeutet, der gegebenenfalls durch Fluor, Chlor, Phenyl oder einen 5- bis 7-gliedrigen, gesättigten oder ungesättigten Heterocyclus mit bis zu 2 Heteroatomen aus der Reihe O, S, N substituiert ist, die ihrerseits durch Fluor, Chlor, geradkettiges oder verzweigtes Alkyl oder Alkoxy mit jeweils bis zu 4 Kohlenstoffatomen, Trifluormethyl oder Trifluormethoxy substituiert sein können,
- D: eine direkte Bindung oder ein Sauerstoffatom bedeutet,
- R¹³: Wasserstoff, Phenyl oder Pyridyl bedeutet, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind, oder einen cyclischen, geradkettigen oder verzweigten, gesättigten oder ungesättigten Kohlenwasserstoffrest mit bis zu 8 Kohlenstoffatomen bedeutet, der gegebenenfalls bis zu 2-fach gleich oder verschieden durch Sauerstoff oder durch -CO-, -CO-NH-, -O-CO-, -CO-O-, -NH-CO-, -SO₂-NH-, -NH-SO₂-, -S(O)ₑ- oder -NR¹⁶ unterbrochen ist,
worin
e die oben angegebene Bedeutung von d hat und mit dieser gleich oder verschieden ist,
R¹⁶ die oben angegebene Bedeutung von R¹⁵ hat und mit dieser gleich oder verschieden ist,
oder der Kohlenwasserstoffrest gegebenenfalls durch Phenyliden oder heterocyclische Reste der Formeln 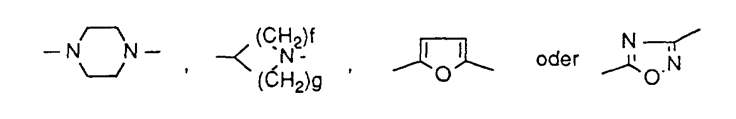 unterbrochen ist,
worin
f und g gleich oder verschieden sind und eine Zahl 1 oder 2 bedeuten,
und der Kohlenwasserstoffrest gegebenenfalls bis zu 2-fach gleich oder verschieden durch Cyclopropyl, Cyclobutyl, Cyclopentyl, Cyclohexyl, Fluor, Chlor, Nitro, Cyano, Hydroxy, -O-NO₂, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, die ihrerseits bis zu 2-fach gleich oder verschieden durch Fluor, Chlor, Cyano oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sein können,
oder der Kohlenwasserstoffrest durch eine Gruppe der Formel -CO₂-R¹⁷, -CONR¹⁸R¹⁹ oder -NR²⁰R²¹ substituiert sein kann,
worin
- R¹⁷: die oben angegebene Bedeutung von R¹⁵ hat und mit dieser gleich oder verschieden ist
und
- R¹⁸, R¹⁹, R²⁰ und R²¹: die oben angegebene Bedeutung von R¹¹ und R¹² haben und mit diesen gleich oder verschieden sind,
und
- R³: für Phenyl steht, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen oder Hydroxy substituiert ist, oder
für Thienyl oder Pyridyl steht, die gegebenenfalls durch Fluor, Chlor, Brom substituiert sind,
und deren Salze.

Besonders bevorzugt sind Verbindungen der allgemeinen Formel (I),
in welcher
- A: für die -CH₂-Gruppe steht,
- R¹: für Wasserstoff, Amino, Cyano, Formyl, Trifluormethyl oder für geradkettiges oder verzweigtes Alkyl mit bis zu 3 Kohlenstoffatomen steht, das gegebenenfalls durch Hydroxy oder durch eine Gruppe der Formel -NR⁶R⁷, -O-CO-R⁸, -O-(CH₂)ₐ-OR^{8'} oder -O-(CH₂)_{b'}-NR⁹R¹⁰ substituiert ist,
worin
R⁶, R⁷, R⁹ und R¹⁰ gleich oder verschieden sind und Wasserstoff, Methyl oder Ethyl bedeuten,
R⁸ und R^{8'} gleich oder verschieden sind und Methyl oder Ethyl bedeuten, und
a und b gleich oder verschieden sind und eine Zahl 2 oder 3 bedeuten,
- R²: für eine Gruppe der Formel -CO-NR¹¹R¹² oder -CO-D-R¹³ steht,
worin
R¹¹ und R¹² gleich oder verschieden sind und Wasserstoff, einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor, Phenyl oder Pyridyl substituiert ist, oder
Phenyl oder Pyridyl bedeuten, die gegebenenfalls durch Fluor, Chlor, Methyl oder Methoxy substituiert sind,
oder
- R¹¹ und R¹²: gemeinsam unter Einbezug des Stickstoffatoms einen 5- bis 6-gliedrigen, ungesättigten Heterocyclus bilden, der gegebenenfalls durch Sauerstoff oder Schwefel oder den Rest NR¹⁵ unterbrochen ist,
worin
R¹⁵ Wasserstoff oder Phenyl bedeutet, das gegebenenfalls durch Fluor, Chlor, Methyl, Methoxy oder Trifluormethyl substituiert ist, oder
einen cyclischen, gesättigten oder ungesättigten, geradkettigen oder verzweigten Kohlenwasserstoffrest mit bis zu 4 Kohlenstoffatomen bedeutet, der gegebenenfalls durch Phenyl oder Pyridyl substituiert ist,
- D: eine direkte Bindung oder ein Sauerstoffatom bedeutet,
- R¹³: Wasserstoff oder Phenyl bedeutet, das gegebenenfalls durch Fluor, Chlor, Methyl, Methoxy oder Trifluormethyl substituiert ist, oder
einen cyclischen, geradkettigen oder verzweigten, gesättigten oder ungesättigten Kohlenwasserstoffrest mit bis zu 6 Kohlenstoffatomen bedeutet, der gegebenenfalls durch Sauerstoff oder Schwefel oder durch -CO-, -CO-NH-, -O-CO-, -CO-O-, -NH-CO-, -SO₂-NH-, -NH-SO₂- oder -NR¹⁶ unterbrochen ist,
worin
R¹⁶ die oben angegebene Bedeutung von R¹⁵ hat und mit dieser gleich oder verschieden ist,
oder der Kohlenwasserstoffrest gegebenenfalls durch Phenyliden oder heterocyclische Reste der Formeln 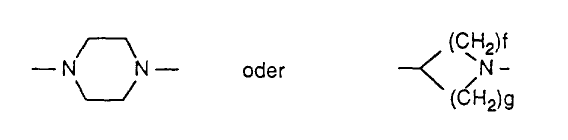 unterbrochen ist,
worin
f und g gleich oder verschieden sind und eine Zahl 1 oder 2 bedeuten,
und der Kohlenwasserstoffrest gegebenenfalls durch Cyclopropyl, Cyclobutyl, Cyclopentyl, Cyclohexyl, Fluor, Chlor, Nitro, Hydroxy, geradkettiges oder verzweigtes Alkylthio oder Alkoxy mit jeweils bis zu 5 Kohlenstoffatomen oder durch Phenyl, Phenoxy, Phenylthio oder Pyridyl substituiert ist, die ihrerseits durch Fluor, Chlor, Methyl, Methoxy, Methylthio substituiert sein können, oder
durch eine Gruppe der Formel -CO₂-R¹⁷, -CONR¹⁸R¹⁹ oder -NR²⁰R²¹ substituiert sein kann,
worin
- R¹⁷: die oben angegebene Bedeutung von R¹⁵ hat und mit dieser gleich oder verschieden ist
und
- R¹⁸, R¹⁹, R²⁰ und R²¹: die oben angegebene Bedeutung von R¹¹ und R¹² haben und mit diesen gleich oder verschieden sind,
und
- R³: für Phenyl steht, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, oder für
Thienyl steht,das gegebenenfalls durch Fluor oder Chlor substituiert ist,
und deren Salze.

Ganz besonders bevorzugt sind Verbindungen der allgemeinen Formel (I),
in welcher
- A: für die CH₂-Gruppe steht,
- R¹: für Wasserstoff, Amino oder Methyl steht,
- R²: für eine Gruppe der Formel -CO-NR¹¹R¹² oder -CO-D-R¹³ steht,
worin
R¹¹ und R¹² gleich oder verschieden sind und Wasserstoff oder einen geradkettigen, verzweigten oder cyclischen Alkylrest mit bis zu 6 Kohlenstoffatomen bedeuten,
D ein Sauerstoffatom bedeutet,
R¹³ Wasserstoff oder geradkettiges oder verzweigtes Alkyl mit bis zu 6 Kohlenstoffatomen bedeutet, das gegebenenfalls durch Cyclopropyl, Cyclobutyl, Cyclopentyl, Cyclohexyl, geradkettiges oder verzweigtes Alkoxy oder Alkylthio mit jeweils bis zu 4 Kohlenstoffatomen, Fluor, Phenyl, Phenoxy oder durch NR²⁰R²¹ substituiert ist,
worin
R²⁰ und R²¹ jeweils für Wasserstoff, Alkyl mit bis zu 4 C-Atomen, das gegebenenfalls durch Phenyl substituiert ist, oder für Phenyl steht, und
R³ für Phenyl steht, das gegebenenfalls durch Fluor, Chlor, Methyl oder Methoxy substituiert ist,
und deren Salze.

Außerdem wurde ein Verfahren zur Herstellung der erfindungsgemäßen Verbindungen der allgemeinen Formel (I) gefunden, dadurch gekennzeichnet,
daß man
[A] Verbindungen der allgemeinen Formel (II) 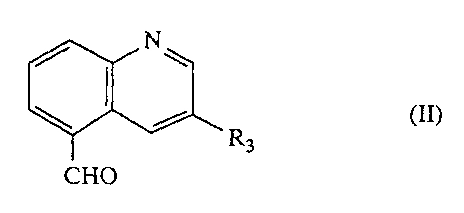 in welcher
   - R³: die oben angegebene Bedeutung hat,
   zunächst mit Acylverbindungen der allgemeinen Formel (III)

   L-CO-CH₂-R² (III)

   in welcher
   - R²: die oben angegebene Bedeutung hat
   und
   - L: die oben angegebene Bedeutung von R¹ hat, wobei im Fall der Hydroxy- und/oder Aminofunktionen, diese gegebenenfalls in geschützter Form vorliegen,
   gegebenenfalls unter Isolierung der Ylidenverbindungen der allgemeinen Formel (IV) 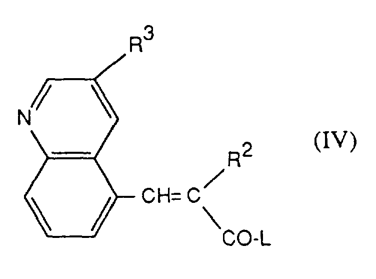 in welcher
   R², R³ und L die oben angegebene Bedeutung haben
   umsetzt, anschließend mit der Verbindung der Formel (V) 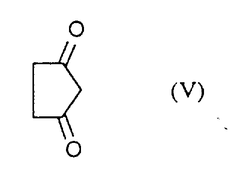 und einer reaktiven Ammoniumverbindung, z.B. Ammoniumacetat, gegebenenfalls unter Isolierung der Zwischenprodukte der allgemeinen Formel (VI) 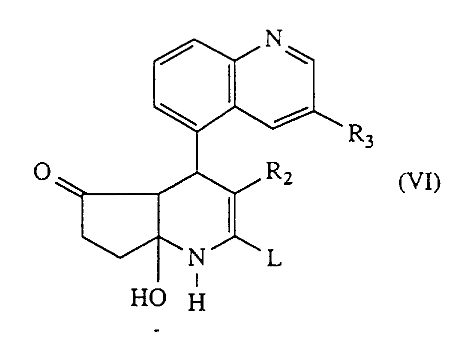 in welcher
   R², R³ und L die oben angegebene Bedeutung haben,
   in inerten Lösemitteln umsetzt,
   und im Fall der Verbindungen der allgemeinen Formel (VI) in einem letzten Schritt, in Anwesenheit eines Hilfsmittels, Wasser abscheidet,
   oder
[B] Verbindungen der allgemeinen Formel (II) direkt mit der Verbindung der Formel (V) und Verbindungen der allgemeinen Formel (VII) 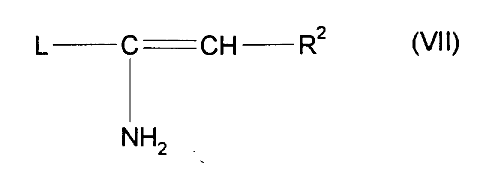 in welcher
   L und R² die oben angegebene Bedeutung haben, umsetzt.

Die erfindungsgemäßen Verfahren können durch folgende Formelschema beispielhaft erläutert werden:

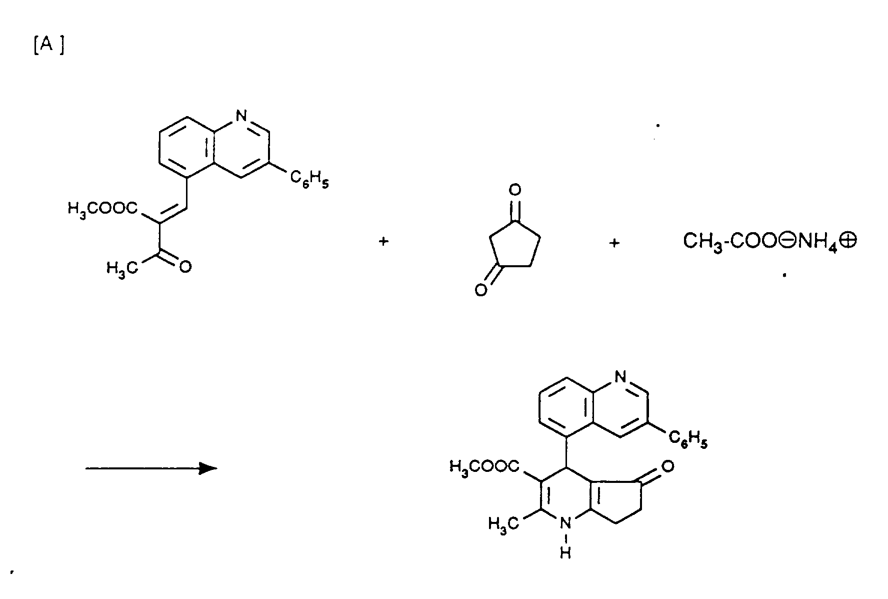

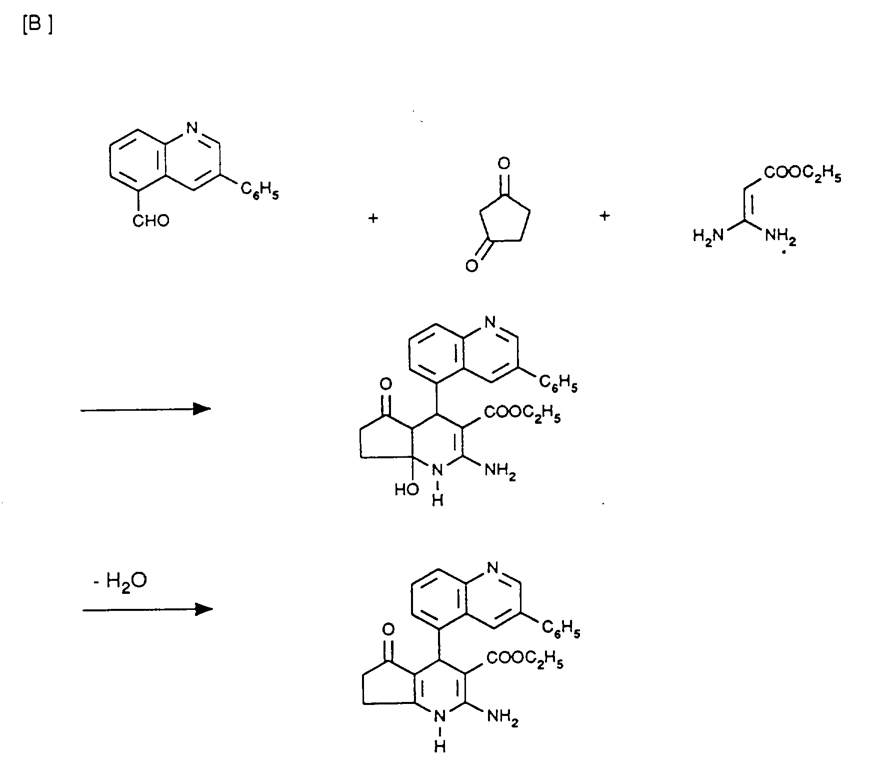

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind in Abhängigkeit von der jeweiligen Verfahrensvariante [A] und [B] Methanol, Isopropanol, Ethanol und n-Propanol, Acetonitril oder Tetrahydrofuran.

Die Reaktionstemperaturen können in einem größeren Bereich variiert werden. Im allgemeinen arbeitet man zwischen +10°C und +150°C, vorzugsweise zwischen +20°C und +100°C. Insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Verbindungen der allgemeinen Formel (II) sind teilweise bekannt und können nach üblichen Methoden hergestellt werden, indem man z.B. die entsprechenden Alkyl- oder Hydroxyalkyl-chinoline oxidiert oder die entsprechenden Carboxychinoline reduziert.

Alternativ kann auch 4-Amino-3-hydroxyphthalid, das durch übliche Hydrierung des literaturbekannten 4-Nitro-3-hydroxyphthalid in Anwesenheit eines Katalysators, bevorzugt mit Palladium/Bariumsulfat, erhalten wird, mit Verbindungen der allgemeinen Formel R³-CH₂-CHO, die teilweise bekannt sind, zu Verbindungen der allgemeinen Formel (II) über die entsprechenden Carbonsäuren umgesetzt werden.

Die Verbindungen der allgemeinen Formel (III), (IV), (V), (VI) und (VII) sind bekannt oder können nach üblichen Methoden hergestellt werden.

Die vorstehenden Herstellungsverfahren sind lediglich zur Verdeutlichung angegeben. Die Herstellung der Verbindungen der Formel (I) sind nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur.
Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (118,5 mmol/l NaCl, 4,75 mmol/l KCl, 1,19 mmol/l KH₂PO₄, 1,19 mmol/l MgSO₄, 25 mmol/l NaHCO₃, 0,013 mmol/l Na₂EDTA), deren CaCl₂-Gehalt 1,2 mmol/l beträgt. Als energielieferndes Substrat werden 10 mmol/l Glucose zugesetzt. vor der Perfusion wird die Lösung partikelfrei filtriert. Die Lösung wird mit Carbogen (95% O₂, 5% CO₂) zur Aufrechterhaltung des pH-Wertes 7,4 begast. Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels eines Druckaufnehmers, der vor dem Herzen mit dem Perfusionssystem in Verbindung steht, registiert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### 2-Amino-4-(3-Phenylchinolin-5-yl)-6-oxo-9-hydroxy- 1,4,5,9-tetrahydro-cyclopentano[1,2-b]pyridin-3-carbonsäureisopropylester

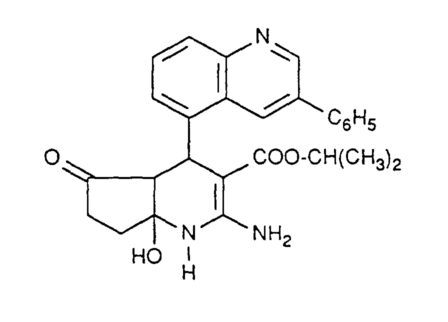

6,99 g (30 mmol) 3-Phenylchinolin-5-carbaldehyd werden in 90 ml Isopropanol mit 2,94 g (30 mmol) Cyclopentan-1,3-dion, 2,46 g (30 mmol) Natriumacetat und 5,4 g (30 mmol) Amidinoessigsäureisopropylester-hydrochlorid versetzt und 7 Stunden zum Rückfluß erhitzt. Das ausgefallene Produkt wird abgesaugt, mit Isopropanol und Wasser gewaschen und getrocknet. Man erhält 4.05 g farblose Kristalle vom Schmelzpunkt 208-209°C.

### Herstellungsbeispiele

### Beispiel 1

### 4-[3-(4-Chlorphenyl)-chinolin-5-yl]-2-methyl-5-oxo-1,4-dihydro-cyclo-pentano-[1,2-b]pyridin-3 -carbonsäureisopropylester

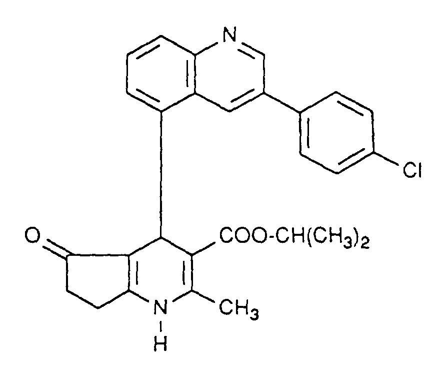

2,67 g (10 mmol) 3-(4-Chlorphenyl)-chinolin-5-carbaldehyd werden in 15 ml Isopropanol und 3,2 ml Essigsäure mit 0,98 g (10 mmol) Cyclopentandion und 1,43 g (10 mmol) 3-Aminocrotonsäureisopropylester 24 Stunden zum Rückfluß gekocht. Es wird abgekühlt und eingeengt. Es wird in Dichlormethan aufgenommen, mit Wasser, Natriumhydrogencarbonatlösung und wieder Wasser gewaschen, getrocknet und eingeengt. Nach Umkristallisation aus Essigester erhält man 1,4 g farblose Kristalle vom Schmelzpunkt 265-266°C.

### Beispiel 2

### 2-Amino-4-(3-phenylchinolin-5-yl)-5-oxo-1,4-dihydro-cyclopentano[1,2-b]pyridin-3-carbonsäureisopropylester

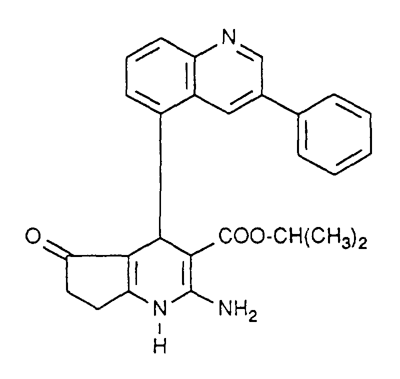

2 g der Verbindung aus Beispiel I in 30 ml DMF werden mit 2 ml Trimethylsilylimidazol versetzt und unter Argon-Atmosphäre langsam auf 90°C erwärmt. Nach 2 Stunden wird mit weiteren 2 ml Trimethylsilylimidazol versetzt und 2 Stunden nachgerührt. Es wird abgekühlt, auf Wasser gegossen, 2 mal mit Essigester extrahiert, die vereinten Essigesterphasen werden mit Wasser gewaschen, getrocknet und eingeengt. Das erhaltene Rohprodukt wird über eine Kieselgelsäule mit Toluol/Aceton-Gemischen gereinigt. Die sauberen Fraktionen werden vereint und eingeengt. Der erhaltene Rückstand wird mit Acetonitril kristallisiert, abgesaugt und mit Acetonitril gewaschen. Man erhält 560 mg einer farblosen Substanz vom Schmelzpunkt 187-189°C.

In Analogie zur Vorschrift der Beispiele 1 und 2 werden die in Tabelle 1 aufgeführten Verbindungen hergestellt:

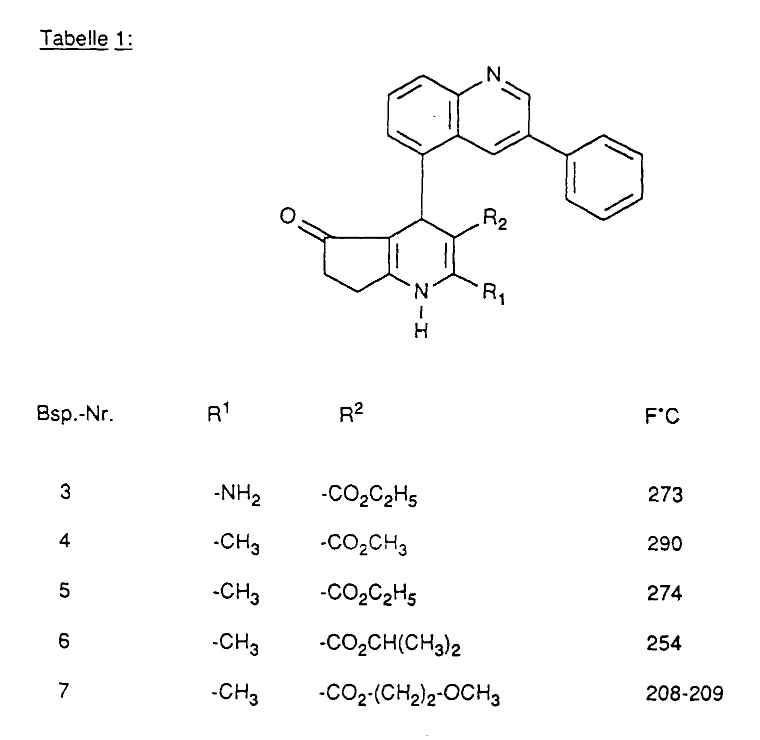

## Patentansprüche

1. 4-Chinolyl-dihydropyridine der allgemeinen Formel (I) 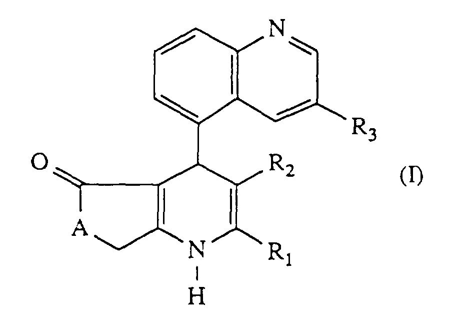 in welcher
A für die -CH₂-Gruppe steht,
R¹ für Wasserstoff, Amino, Cyano, Formyl, Trifluormethyl oder für geradkettiges oder verzweigtes Alkyl mit bis zu 8 Kohlenstoffatomen steht, das gegebenenfalls durch Hydroxy oder durch eine Gruppe der Formel -NR⁶R⁷, -O-CO-R⁸, -O-(CH₂)ₐ-OR^{8'} oder -O-(CH₂)_{b'}-NR⁹R¹⁰ substituiert ist,
worin
R⁶, R⁷, R⁹ und R¹⁰ gleich oder verschieden sind und Wasserstoff, Phenyl oder geradkettiges oder verzweigtes Alkyl mit bis zu 6 Kohlenstoffatomen bedeuten,
R⁸ und R^{8'} gleich oder verschieden sind und geradkettiges oder verzweigtes Alkyl mit bis zu 6 Kohlenstoffatomen bedeuten,
und
a und b gleich oder verschieden sind und eine Zahl 2, 3, 4 oder 5 bedeuten,
R² für eine Gruppe der Formel -CO-NR¹¹R¹² oder -CO-D-R¹³ steht,
worin
R¹¹ und R¹² gleich oder verschieden sind und Wasserstoff, einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
oder
R¹¹ und R¹² gemeinsam unter Einbezug des Stickstoffatoms einen 3- bis 8-gliedrigen, gesättigen oder ungesättigten Heterocyclus bilden, der gegebenenfalls durch ein Sauerstoffatom oder durch einen Rest der Formel S(O)_{d}, -CO- oder -NR¹⁵ unterbrochen ist,
worin
d eine Zahl 0, 1 oder 2 bedeutet,
R¹⁵ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl mit jeweils bis zu 8 Kohlenstoffatomen oder durch Halogenalkyl, Halogenalkoxy oder Halogenalkylthio mit jeweils bis zu 4 Kohlenstoffatomen substituiert ist, oder
einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,
und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das seinerseits durch Aryl mit 6 bis 10 Kohlenstoffatomen substituiert sein kann,
D eine direkte Bindung oder ein Sauerstoffatom bedeutet,
R¹³ Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, wobei die Cyclen gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder
einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch -CO-, -CO-NH-, -O-CO-, -CO-O-, -NH-CO-, -SO₂-NH-, -NH-SO₂-, -S(O)ₑ-oder -NR¹⁶ unterbrochen ist,
worin
e die oben angegebene Bedeutung von d hat und mit dieser gleich oder verschieden ist,
R¹⁶ die oben angegebene Bedeutung von R¹⁵ hat und mit dieser gleich oder verschieden ist,
oder der Kohlenstoffrest gegebenenfalls bis zu 3-fach gleich oder verschieden durch Aryliden mit 6 bis 10 Kohlenstoffatomen oder heterocyclische Reste der Formeln 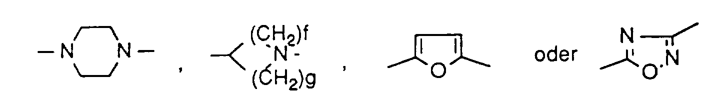 unterbrochen ist,
worin
f und g gleich oder verschieden sind und eine Zahl 1 oder 2 bedeuten,
und wobei die Aryl- und Hetero-Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,
und der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, -O-NO₂, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, wobei die Cyclen ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
der Kohlenwasserstoffrest durch eine Gruppe der Formel -CO₂-R¹⁷, -CONR¹⁸R¹⁹ oder -NR²⁰R²¹ substituiert sein kann,
worin
R¹⁷ die oben angegebene Bedeutung von R¹⁵ hat und mit dieser gleich oder verschieden ist
und
R¹⁸, R¹⁹, R²⁰ und R²¹ die oben angegebene Bedeutung von R¹¹ und R¹² haben und mit diesen gleich oder verschieden sind,
und
R³ für Aryl mit 6 bis 10 Kohlenstoffatomen steht, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder für Thienyl oder Pyridyl steht, die gegebenenfalls durch Halogen substituiert sind
und deren Salze.

2. Verbindungen der allgemeinen Formel (I) gemäß Anspruch 1, in welcher
A für die -CH₂-Gruppe steht,
R¹ für Wasserstoff, Amino, Cyano, Formyl, Trifluormethyl oder für geradkettiges oder verzweigtes Alkyl mit bis zu 6 Kohlenstoffatomen steht, das gegebenenfalls durch Hydroxy oder durch eine Gruppe der Formel -NR⁶R⁷, -O-CO-R⁸, -O-(CH₂)ₐ-OR^{8'} oder -O-(CH₂)_{b}, -NR⁹R¹⁰ substituiert ist,
worin
R⁶, R⁷, R⁹ und R¹⁰ gleich oder verschieden sind und Wasserstoff oder geradkettiges oder verzweigtes Alkyl mit bis zu 4 Kohlenstoffatomen bedeuten,
R⁸ und R^{8'} gleich oder verschieden sind und geradkettiges oder verzweigtes Alkyl mit bis zu 4 Kohlenstoffatomen bedeuten,
und
a und b gleich oder verschieden sind und eine Zahl 2, 3 oder 4 bedeuten,
R² für eine Gruppe der Formel -CO-NR¹¹R¹² oder -CO-D-R¹³ steht,
worin
R¹¹ und R¹² gleich oder verschieden sind und Wasserstoff, einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor, Hydroxy, Cyano oder durch Phenyl, Phenyloxy, Phenylthio oder Pyridyl substituiert ist, wobei die Cyclen ihrerseits durch Fluor, Chlor oder durch Alkyl, Alkoxy, Alkoxycarbonyl mit jeweils bis zu 2 Kohlenstoffatomen, Trifluormethyl oder Trifluormethoxy substituiert sein können, oder Phenyl oder Pyridyl bedeuten, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind,
oder
R¹¹ und R¹² gemeinsam unter Einbezug des Stickstoffatoms einen 5- bis 7-gliedrigen, gesättigen oder ungesättigten Heterocyclus bilden, der gegebenenfalls durch Sauerstoffatom oder durch einen Rest der Formel S(O)_{d}, -CO- oder -NR¹⁵ unterbrochen ist,
worin
d eine Zahl 0, 1 oder 2 bedeutet,
R¹⁵ Wasserstoff oder Phenyl bedeutet, das gegebenenfalls durch Fluor, Chlor, durch geradkettiges oder verzweigtes Alkyl oder Alkoxy mit jeweils bis zu 4 Kohlenstoffatomen, Trifluormethyl oder Trifluormethoxy substituiert ist, oder
einen cyclischen, gesättigten oder ungesättigten, geradkettigen oder verzweigten Kohlenwasserstoffrest mit bis zu 4 Kohlenstoffatomen bedeutet, der gegebenenfalls durch Fluor, Chlor, Phenyl oder einen 5- bis 7-gliedrigen, gesättigten oder ungesättigten Heterocyclus mit bis zu 2 Heteroatomen aus der Reihe O, S, N substituiert ist, die ihrerseits durch Fluor, Chlor, geradkettiges oder verzweigtes Alkyl oder Alkoxy mit jeweils bis zu 4 Kohlenstoffatomen, Trifluormethyl oder Trifluormethoxy substituiert sein können,
D eine direkte Bindung oder ein Sauerstoffatom bedeutet,
R¹³ Wasserstoff, Phenyl oder Pyridyl bedeutet, die gegebenenfalls durch Fluor, Chlor oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sind, oder
einen cyclischen, geradkettigen oder verzweigten, gesättigten oder ungesättigten Kohlenwasserstoffrest mit bis zu 8 Kohlenstoffatomen bedeutet, der gegebenenfalls bis zu 2-fach gleich oder verschieden durch ein Sauerstoff oder durch -CO-, -CO-NH-, -O-CO-, -CO-O-, -NH-CO-, -SO₂-N-, -NH-SO₂-, -S(O)ₑ- oder NR¹⁶ unterbrochen ist,
worin
e die oben angegebene Bedeutung von d hat und mit dieser gleich oder verschieden ist,
R¹⁶ die oben angegebene Bedeutung von R¹⁵ hat und mit dieser gleich oder verschieden ist,
oder der Kohlenwasserstoffrest gegebenenfalls durch Phenyliden oder heterocyclische Reste der Formeln 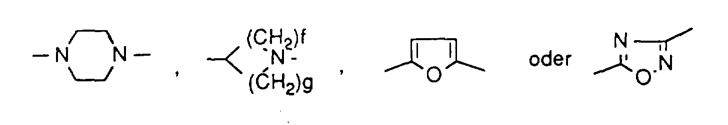 unterbrochen ist,
worin
f und g gleich oder verschieden sind und eine Zahl 1 oder 2 bedeuten,
und der Kohlenwasserstoffrest gegebenenfalls bis zu 2-fach gleich oder verschieden durch Cyclopropyl, Cyclobutyl, Cyclopentyl, Cyclohexyl, Fluor, Chlor, Nitro, Cyano, Hydroxy, -O-NO₂, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, die ihrerseits bis zu 2-fach gleich oder verschieden durch Fluor, Chlor, Cyano oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sein können,
oder der Kohlenwasserstoffrest durch eine Gruppe der Formel -CO₂-R¹⁷, -CONR¹⁸R¹⁹ oder -NR²⁰R²¹ substituiert sein kann,
worin
R¹⁷ die oben angegebene Bedeutung von R¹⁵ hat und mit dieser gleich oder verschieden ist
und
R¹⁸, R¹⁹, R²⁰ und R²¹ die oben angegebene Bedeutung von R¹¹ und R¹² haben und mit diesen gleich oder verschieden sind,
und
R³ für Phenyl steht, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen oder Hydroxy substituiert ist, oder
für Thienyl oder Pyridyl steht, die gegebenenfalls durch Fluor, Chlor, Brom substituiert sind,
und deren Salze.

3. Verbindungen der allgemeinen Formel (I) gemäß Anspruch 1, in welcher
A für die -CH₂-Gruppe steht,
R¹ für Wasserstoff, Amino, Cyano, Formyl, Trifluormethyl oder für geradkettiges oder verzweigtes Alkyl mit bis zu 3 Kohlenstoffatomen steht, das gegebenenfalls durch Hydroxy oder durch eine Gruppe der Formel -NR⁶R⁷, -O-CO-R⁸, -O-(CH₂)ₐ-OR^{8'} oder -O-(CH₂)_{b'}-NR⁹R¹⁰ substituiert ist,
worin
R⁶, R⁷, R⁹ und R¹⁰ gleich oder verschieden sind und Wasserstoff, Methyl oder Ethyl bedeuten,
R⁸ und R^{8'} gleich oder verschieden sind und Methyl oder Ethyl bedeuten,
und
a und b gleich oder verschieden sind und eine Zahl 2 oder 3 bedeuten,
R² für eine Gruppe der Formel -CO-NR¹¹R¹² oder -CO-D-R¹³ steht,
worin
R¹¹ und R¹² gleich oder verschieden sind und Wasserstoff, einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor, Phenyl oder Pyridyl substituiert ist, oder Phenyl oder Pyridyl bedeuten, die gegebenenfalls durch Fluor, Chlor, Methyl oder Methoxy substituiert sind,
oder
R¹¹ und R¹² gemeinsam unter Einbezug des Stickstoffatoms einen 5- bis 6-gliedrigen, ungesättigten Heterocyclus bilden, der gegebenenfalls durch Sauerstoff oder Schwefel oder den Rest NR¹⁵ unterbrochen ist,
worin
R¹⁵ Wasserstoff oder Phenyl bedeutet, das gegebenenfalls durch Fluor, Chlor, Methyl, Methoxy oder Trifluormethyl substituiert ist, oder
einen cyclischen, gesättigten oder ungesättigten, geradkettigen oder verzweigten Kohlenwasserstoffrest mit bis zu 4 Kohlenstoffatomen bedeutet, der gegebenenfalls durch Phenyl oder Pyridyl substituiert ist,
D eine direkte Bindung oder ein Sauerstoffatom bedeutet,
R¹³ Wasserstoff oder Phenyl bedeutet, das gegebenenfalls durch Fluor, Chlor, Methyl, Methoxy oder Trifluormethyl substituiert ist, oder
einen cyclischen, geradkettigen oder verzweigten, gesättigten oder ungesättigten Kohlenwasserstoffrest mit bis zu 6 Kohlenstoffatomen bedeutet, der gegebenenfalls durch Sauerstoff oder Schwefel oder durch -CO-, -CO-NH-, -O-CO-, -CO-O-, -NH-CO-, -SO₂-NH-, -NH-SO₂- oder -NR¹⁶ unterbrochen ist,
worin
R¹⁶ die oben angegebene Bedeutung von R¹⁵ hat und mit dieser gleich oder verschieden ist,
oder der Kohlenwasserstoffrest gegebenenfalls durch Phenyliden oder heterocyclische Reste der Formeln 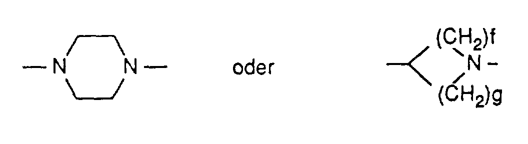 unterbrochen ist,
worin
f und g gleich oder verschieden sind und eine Zahl 1 oder 2 bedeuten,
und der Kohlenwasserstoffrest gegebenenfalls durch Cyclopropyl, Cyclobutyl, Cyclopentyl, Cyclohexyl, Fluor, Chlor, Nitro, Hydroxy, geradkettiges oder verzweigtes Alkylthio oder Alkoxy mit jeweils bis zu 5 Kohlenstoffatomen oder durch Phenyl, Phenoxy, Phenylthio oder Pyridyl substituiert ist, die ihrerseits durch Fluor, Chlor, Methyl, Methoxy, Methylthio substituiert sein können, oder
durch eine Gruppe der Formel -CO₂-R¹⁷, -CONR¹⁸R¹⁹ oder -NR²⁰R²¹ substituiert sein kann,
worin
R¹⁷ die oben angegebene Bedeutung von R¹⁵ hat und mit dieser gleich oder verschieden ist
und
R¹⁸, R¹⁹, R²⁰ und R²¹ die oben angegebene Bedeutung von R¹¹ und R¹² haben und mit diesen gleich oder verschieden sind,
und
R³ für Phenyl steht, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, oder für Thienyl steht,das gegebenenfalls durch Fluor oder Chlor substituiert ist,
und deren Salze.

4. Verbindungen der allgemeinen Formel (1) gemäß Anspruch 1, in welcher
A für die -CH₂-Gruppe steht,
R¹ für Wasserstoff, Amino oder Methyl steht,
R² für eine Gruppe der Formel -CO-NR¹¹R¹² oder -CO-D-R¹³ steht,
worin
R¹¹ und R¹² gleich oder verschieden sind und Wasserstoff oder einen geradkettigen, verzweigten oder cyclischen Alkylrest mit bis zu 6 Kohlenstoffatomen bedeuten,
D ein Sauerstoffatom bedeutet,
R¹³ Wasserstoff oder geradkettiges oder verzweigtes Alkyl mit bis zu 6 Kohlenstoffatomen bedeutet, das gegebenenfalls durch Cyclopropyl, Cyclobutyl, Cyclopentyl, Cyclohexyl, geradkettiges oder verzweigtes Alkoxy oder Alkylthio mit jeweils bis zu 4 Kohlenstoffatomen, Fluor, Phenyl, Phenoxy oder durch NR²⁰R²¹ substituiert ist,
worin
R²⁰ und R²¹ jeweils für Wasserstoff, Alkyl mit bis zu 4 C-Atomen, das gegebenenfalls durch Phenyl substituiert ist, oder für Phenyl steht, und
R³ für Phenyl steht, das gegebenenfalls durch Fluor, Chlor, Methyl oder Methoxy substituiert ist,
und deren Salze.

5. Verfahren zur Herstellung von Verbindungen der allgemeinen Formel (I) 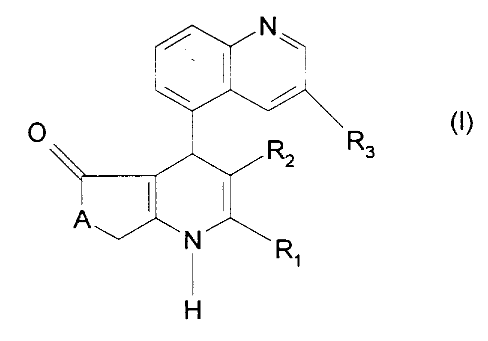 in welcher A, R¹, R² und R³ die in Anspruch 1 angegebene Bedeutung haben, und deren Salze, dadurch gekennzeichnet, daß man
[A] Verbindungen der allgemeinen Formel (II) 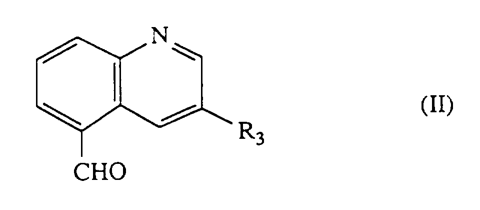 in welcher
R³ die oben angegebene Bedeutung hat,
zunächst mit Acylverbindungen der allgemeinen Formel (III)
L-CO-CH₂-R² (III)
in welcher
R² die oben angegebene Bedeutung hat
und
L die oben angegebene Bedeutung von R¹ hat, wobei im Fall der Hydroxy- und/oder Aminofunktionen, diese gegebenenfalls in geschützter Form vorliegen,
gegebenenfalls unter Isolierung der Ylidenverbindungen der allgemeinen Formel (IV) 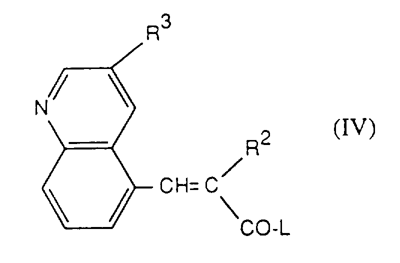 in welcher
R², R³ und L die oben angegebene Bedeutung haben
umsetzt, anschließend mit der Verbindung der Formel (V) 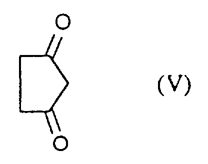 und einer reaktiven Ammoniumverbindung, gegebenenfalls unter Isolierung der Zwischenprodukte der allgemeinen Formel (VI) 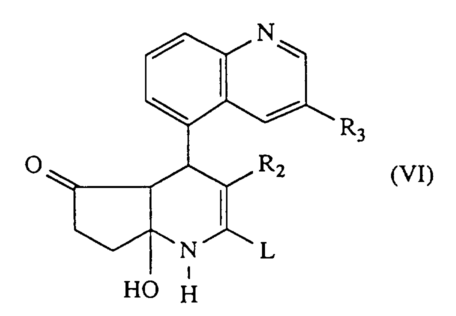 in welcher R², R³ und L die oben angegebene Bedeutung haben, in inerten Lösemitteln umsetzt, und im Fall der Verbindungen der allgemeinen Formel (VI) in einem letzten Schritt, in Anwesenheit eines Hilfsmittels, Wasser abscheidet, oder
[B] Verbindungen der allgemeinen Formel (II) direkt mit der Verbindung der Formel (V) und Verbindungen der allgemeinen Formel (VII) 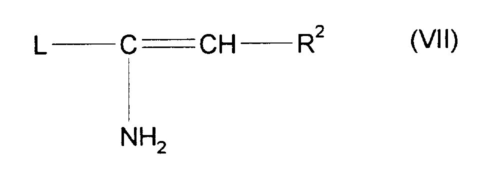 in welcher L und R² die oben angegebene Bedeutung haben, umsetzt.

6. Verbindungen der allgmeinen Formel (I) gemäß Anspruch 1 zur Verwendung bei der Behandlung von Herz-Kreislauferkrartkungen.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfs und Trägerstoffen in eine geeignete Applikationsform überführt.

9. Verwendung von Verbindungen der allgemeinen Formel (I) gemäß Anspruch 1 in Arzneimitteln mit positiv ionotroper Wirkung.

## Claims

1. 4-Quinolyl-dihydropyridines of the general formula (I) 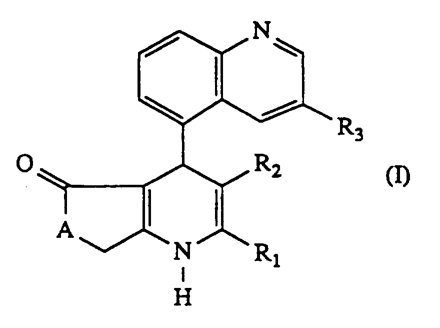 in which
A represents the CH₂ group,
R¹ represents hydrogen, amino, cyano, formyl or trifluoromethyl, or represents straight-chain or branched alkyl having up to 8 carbon atoms, which is optionally substituted by hydroxyl or by a group of the formula -NR⁶R⁷, -O-CO-R⁸, -O-(CH₂)ₐ-OR^{8'} or -O-(CH₂)_{b}, -NR⁹R¹⁰,
in which
R⁶, R⁷, R⁹ and R¹⁰ are identical or different and denote hydrogen, phenyl or straight-chain or branched alkyl having up to 6 carbon atoms,
R⁸ and R^{8'} are identical or different and denote straight-chain or branched alkyl having up to 6 carbon atoms,
and
a and b are identical or different and denote a number 2, 3, 4 or 5,
R² represents a group of the formula -CO-NR¹¹R¹² or -CO-D-R¹³,
in which
R¹¹ and R¹² are identical or different and denote hydrogen, a straight-chain, branched, cyclic, saturated or unsaturated hydrocarbon radical having up to 8 carbon atoms, which is optionally substituted by halogen, hydroxyl or cyano, or by aryl, aryloxy or arylthio having in each case 6 to 10 carbon atoms, or by a 5- to 7-membered, saturated or unsaturated heterocycle having up to 3 heteroatoms selected from the group comprising S, N or 0, where the cycles, for their part, can be substituted by halogen or cyano, or by straight-chain or branched alkyl, alkoxy, alkylthio, alkoxycarbonyl, halogenoalkyl, halogenoalkoxy or halogenoalkylthio having in each case up to 4 carbon atoms, or aryl having 6 to 10 carbon atoms or a 5- to 7-membered, saturated or unsaturated heterocycle having up to 3 heteroatoms selected from the group comprising S, N or O, which are optionally substituted identically or differently up to 2 times by halogen or cyano, or by straight-chain or branched alkyl, alkoxy, alkylthio, alkoxycarbonyl, halogenoalkyl, halogenoalkoxy or halogenoalkylthio having in each case up to 4 carbon atoms,
or
R¹¹ and R¹², together and with the inclusion of the nitrogen atom, form a 3- to 8-membered, saturated or unsaturated heterocycle, which is optionally interrupted by an oxygen atom or by a radical of the formula S(O)_{d}, -CO- or -NR¹⁵,
in which
d denotes a number 0, 1 or 2,
R¹⁵ denotes hydrogen or aryl having 6 to 10 carbon atoms, which is optionally substituted identically or differently up to 2 times by halogen or cyano, or by straight-chain or branched alkyl, alkoxy, alkylthio or alkoxycarbonyl having in each case up to 8 carbon atoms, or by halogenoalkyl, halogenoalkoxy or halogenoalkylthio having in each case up to 4 carbon atoms, or
denotes a cyclic, saturated or unsaturated, straight-chain or branched hydrocarbon radical having up to 8 carbon atoms, which is optionally substituted by hydroxyl or halogen, or by aryl having 6 to 10 carbon atoms, or by a 5- to 7-membered, saturated or unsaturated heterocycle having up to 3 heteroatoms selected from the group comprising S, N or 0, where the cycles, for their part, can be substituted identically or differently up to 2 times by halogen or cyano, or by straight-chain or branched alkyl, alkoxy, alkylthio, alkoxycarbonyl, halogenoalkyl, halogenoalkoxy or halogenoalkylthio having in each case up to 4 carbon atoms,
and the heterocycle is optionally substituted by straight-chain or branched alkoxy or alkylthio having in each case up to 4 carbon atoms, by halogen, by aryl having 6 to 10 carbon atoms, by a 5- to 7-membered, saturated or unsaturated heterocycle having up to 3 heteroatoms selected from the group comprising S, N or O, or by straight-chain or branched alkyl having up to 4 carbon atoms which, for its part, can be substituted by aryl having 6 to 10 carbon atoms,
D denotes a direct linkage or an oxygen atom,
R¹³ denotes hydrogen or aryl having 6 to 10 carbon atoms or a 5- to 7-membered, saturated or unsaturated heterocycle having up to 3 heteroatoms selected from the group comprising S, N or 0, where the cycles are optionally substituted identically or differently up to 3 times by halogen or cyano, or by straight-chain or branched alkyl, alkoxy, alkylthio, alkoxycarbonyl, halogenoalkyl, halogenoalkoxy or halogenoalkylthio having in each case up to 4 carbon atoms, or
denotes a cyclic, straight-chain or branched, saturated or unsaturated hydrocarbon radical having up to 10 carbon atoms, which is optionally interrupted identically or differently up to 3 times by oxygen or by -CO-, -CO-NH-, -O-CO-, -CO-O-, -NH-CO-, -SO₂-NH-, -NH-SO₂-, -S(O)ₑ- or -NR¹⁶,
in which
e has the abovementioned meaning of d and is identical to or different from the latter,
R¹⁶ has the abovementioned meaning of R¹⁵ and is identical to or different from the latter,
or the carbon radical is optionally interrupted identically or differently up to 3 times by arylidene having 6 to 10 carbon atoms or by heterocyclic residues of the formulae 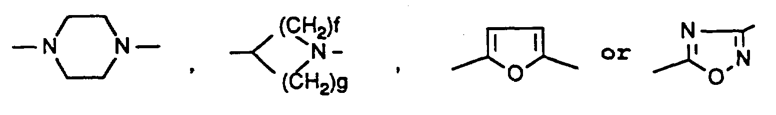 in which,
f and g are identical or different and denote a number 1 or 2,
and where the aryl and hetero cycles, for their part, can be substituted by halogen or cyano, or by straight-chain or branched alkyl, alkoxy, alkylthio, alkoxycarbonyl, halogenoalkyl, halogenoalkoxy or halogenoalkylthio having in each case up to 4 carbon atoms,
and the hydrocarbon radical is optionally substituted identically or differently up to 3 times by cycloalkyl having 3 to 8 carbon atoms, by halogen, nitro, cyano, hydroxyl or -O-NO₂, or by straight-chain or branched alkylthio, alkoxy or acyloxy having in each case up to 8 carbon atoms, or by aryl, aryloxy or arylthio having in each case 6 to 10 carbon atoms, or by a 5- to 7-membered, saturated or unsaturated heterocycle having up to 3 heteroatoms selected from the group comprising S, N or O, where the cycles, for their part, can be substituted identically or differently up to 3 times by halogen or cyano, or by straight-chain or branched alkyl, alkoxy, alkylthio, alkoxycarbonyl, halogenoalkyl, halogenoalkoxy or halogenoalkylthio having in each case up to 4 carbon atoms, or
the hydrocarbon radical can be substituted by a group of the formula -CO₂-R¹⁷, -CONR¹⁸R¹⁹ or -NR²⁰R₂₁,
in which
R¹⁷ has the abovementioned meaning of R¹⁵ and is identical to or different from the latter
and
R¹⁸, R¹⁹, R²⁰ and R²¹ have the abovementioned meaning of R¹¹ and R¹² and are identical to or different from these latter,
and
R³ represents aryl having 6 to 10 carbon atoms, which is optionally substituted identically or differently up to 2 times by halogen, nitro, cyano, trifluoromethyl, trifluoromethoxy or trifluoromethylthio, or by straight-chain or branched alkyl, alkoxy or alkoxycarbonyl having in each case up to 8 carbon atoms, or by carboxyl,
or represents thienyl or pyridyl which are optionally substituted by halogen
and salts thereof.

2. Compounds of the general formula (I) according to Claim 1, in which
A represents the CH₂ group,
R¹ represents hydrogen, amino, cyano, formyl or trifluoromethyl, or represents straight-chain or branched alkyl having up to 6 carbon atoms, which is optionally substituted by hydroxyl or by a group of the formula -NR⁶R⁷, -O-CO-R⁸, -O-(CH₂)ₐ-OR^{8'} or -O- (CH₂)_{b},-NR⁹R¹⁰,
in which
R⁶, R⁷, R⁹ and R¹⁰ are identical or different and denote hydrogen or straight-chain or branched alkyl having up to 4 carbon atoms,
R⁸ and R^{8'} are identical or different and denote straight-chain or branched alkyl having up to 4 carbon atoms,
and
a and b are identical or different and denote a number 2, 3 or 4,
R² represents a group of the formula -CO-NR¹¹R¹² or -CO-D-R¹³,
in which
R¹¹ and R¹² are identical or different and denote hydrogen, a straight-chain, branched, cyclic, saturated or unsaturated hydrocarbon radical having up to 6 carbon atoms, which is optionally substituted by fluorine, chlorine, hydroxyl or cyano, or by phenyl, phenyloxy, phenylthio or pyridyl, where the cycles, for their part, can be substituted by fluorine or chlorine, or by alkyl, alkoxy or alkoxycarbonyl having in each case up to 2 carbon atoms, or by trifluoromethyl or trifluoromethoxy, or denote phenyl or pyridyl, which are optionally substituted by fluorine or chlorine, or by straight-chain or branched alkyl, alkoxy, alkylthio or alkoxycarbonyl having in each case up to 4 carbon atoms, or by trifluoromethyl or trifluoromethoxy,
or
R¹¹ and R¹², together and with the inclusion of the nitrogen atom, form a 5- to 7-membered, saturated or unsaturated heterocycle, which is optionally interrupted by an oxygen atom or by a radical of the formula S(O)_{d}, -CO- or -NR¹⁵,
in which
d denotes a number 0, 1 or 2,
R¹⁵ denotes hydrogen or phenyl, which is optionally substituted by fluorine or chlorine, or by straight-chain or branched alkyl or alkoxy having in each case up to 4 carbon atoms, or by trifluoromethyl or trifluoromethoxy, or denotes a cyclic, saturated or unsaturated, straight-chain or branched hydrocarbon radical having up to 4 carbon atoms, which is optionally substituted by fluorine or chlorine, or by phenyl or a 5- to 7-membered, saturated or unsaturated heterocycle having up to 2 heteroatoms selected from the group comprising 0, S and N, which, for their part, can be substituted by fluorine or chlorine, or by straight-chain or branched alkyl or alkoxy having in each case up to 4 carbon atoms, or by trifluoromethyl or trifluoromethoxy,
D denotes a direct linkage or an oxygen atom,
R¹³ denotes hydrogen or phenyl or pyridyl, which are optionally substituted by fluorine or chlorine, or by straight-chain or branched alkyl, alkoxy, alkylthio or alkoxycarbonyl having in each case up to 4 carbon atoms, or by trifluoromethyl or trifluoromethoxy, or denotes a cyclic, straight-chain or branched, saturated or unsaturated hydrocarbon radical having up to 8 carbon atoms, which is optionally interrupted identically or differently up to 2 times by oxygen or by -CO-, -CO-NH-, -O-CO-, -CO-O-, -NH-CO-, -SO₂-NH-, -NH-SO₂-, -S(O)ₑ- or -NR¹⁶,
in which
e has the abovementioned meaning of d and is identical to or different from the latter,
R¹⁶ has the abovementioned meaning of R¹⁵ and is identical to or different from the latter,
or the hydrocarbon radical is optionally interrupted by phenylidene or by heterocyclic residues of the formulae 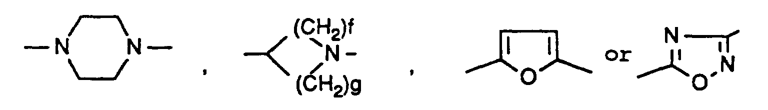 in which,
f and g are identical or different and denote a number 1 or 2,
and the hydrocarbon radical is optionally substituted identically or differently up to 2 times by cyclopropyl, cyclobutyl, cyclopentyl, cyclohexyl, fluorine, chlorine, nitro, cyano, hydroxyl or -O-NO₂, or by straight-chain or branched alkylthio, alkoxy or acyloxy having in each case up to 4 carbon atoms, or by phenyl, phenoxy, phenylthio or pyridyl, which, for their part, can be substituted identically or differently up to 2 times by fluorine, chlorine or cyano, or by straight-chain or branched alkyl, alkoxy, alkylthio or alkoxycarbonyl having in each case up to 4 carbon atoms, or by trifluoromethyl or trifluoromethoxy,
or the hydrocarbon radical can be substituted by a group of the formula -CO₂-R¹⁷, -CONR¹⁸R¹⁹ or -NR²⁰R²¹, in which
R¹⁷ has the abovementioned meaning of R¹⁵ and is identical to or different from the latter
and
R¹⁸, R¹⁹, R²⁰ and R²¹ have the abovementioned meaning of R¹¹ and R¹² and are identical to or different from these latter,
and
R³ represents phenyl, which is optionally substituted by fluorine, chlorine, nitro, cyano or trifluoromethyl, or by straight-chain or branched alkyl or alkoxy having in each case up to 6 carbon atoms, or by hydroxyl, or
represents thienyl or pyridyl, which are optionally substituted by fluorine, chlorine or bromine,
and salts thereof.

3. Compounds of the general formula (I) according to Claim 1, in which
A represents the CH₂ group,
R¹ represents hydrogen, amino, cyano, formyl or trifluoromethyl, or represents straight-chain or branched alkyl having up to 3 carbon atoms, which is optionally substituted by hydroxyl or by a group of the formula -NR⁶R⁷, -O-CO-R⁸, -O-(CH₂)ₐ-OR^{8'} or -O-(CH₂)_{b},-NR⁹R¹⁰,
in which
R⁶, R⁷, R⁹ and R¹⁰ are identical or different and denote hydrogen, methyl or ethyl,
R⁸ and R^{8'} are identical or different and denote methyl or ethyl,
and a and b are identical or different and denote a number 2 or 3,
R² represents a group of the formula -CO-NR¹¹R¹² or -CO-D-R¹³,
in which
R¹¹ and R¹² are identical or different and denote hydrogen, a straight-chain, branched, cyclic, saturated or unsaturated hydrocarbon radical having up to 6 carbon atoms, which is optionally substituted by fluorine, chlorine, phenyl or pyridyl, or denote phenyl or pyridyl, which are optionally substituted by fluorine, chlorine, methyl or methoxy,
or
R¹¹ and R¹², together and with the inclusion of the nitrogen atom, form a 5- to 6-membered, unsaturated heterocycle, which is optionally interrupted by oxygen or sulphur or the radical NR¹⁵,
in which
R¹⁵ denotes hydrogen or phenyl, which is optionally substituted by fluorine, chlorine, methyl, methoxy or trifluoromethyl, or denotes a cyclic, saturated or unsaturated, straight-chain or branched hydrocarbon radical having up to 4 carbon atoms, which is optionally substituted by phenyl or pyridyl,
D denotes a direct linkage or an oxygen atom,
R¹³ denotes hydrogen or phenyl, which is optionally substituted by fluorine, chlorine, methyl, methoxy or trifluoromethyl, or denotes a cyclic, straight-chain or branched saturated or unsaturated hydrocarbon radical having up to 6 carbon atoms, which is optionally interrupted by oxygen or sulphur or by -CO-, -CO-NH-, -O-CO-, -CO-O-, -NH-CO-, -SO₂-NH-, -NH-SO₂- or -NR¹⁶,
in which
R¹⁶ has the abovementioned meaning of R¹⁵ and is identical to or different from the latter,
or the hydrocarbon radical is optionally interrupted by phenylidene or by heterocyclic residues of the formulae 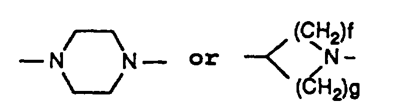 in which,
f and g are identical or different and denote a number 1 or 2,
and the hydrocarbon radical is optionally substituted by cyclopropyl, cyclobutyl, cyclopentyl, cyclohexyl, fluorine, chlorine, nitro or hydroxyl, or by straight-chain or branched alkylthio or alkoxy having in each case up to 5 carbon atoms, or by phenyl, phenoxy, phenylthio or pyridyl, which, for their part, can be substituted by fluorine, chlorine, methyl, methoxy or methylthio, or can be substituted by a group of the formula -CO₂-R¹⁷, -CONR¹⁸R¹⁹ or -NR²⁰R²¹,
in which
R¹⁷ has the abovementioned meaning of R¹⁵ and is identical to or different from the latter
and
R¹⁸, R¹⁹, R²⁰ and R²¹ have the abovementioned meaning of R¹¹ and R¹² and are identical to or different from these latter,
and
R³ represents phenyl, which is optionally substituted by fluorine, chlorine, nitro or trifluoromethyl, or by straight-chain or branched alkyl or alkoxy having in each case up to 4 carbon atoms, or represents
thienyl, which is optionally substituted by fluorine or chlorine,
and salts thereof.

4. Compounds of the general formula (I) according to Claim 1, in which
A represents the CH₂ group,
R¹ represents hydrogen, amino or methyl,
R² represents a group of the formula -CO-NR¹¹R¹² or -CO-D-R¹³,
in which
R¹¹ and R¹² are identical or different and denote hydrogen or a straight-chain, branched or cyclic alkyl radical having up to 6 carbon atoms,
D denotes an oxygen atom,
R¹³ denotes hydrogen or straight-chain or branched alkyl having up to 6 carbon atoms, which is optionally substituted by cyclopropyl, cyclobutyl, cyclopentyl or cyclohexyl, or by straight-chain or branched alkoxy or alkylthio having in each case up to 4 carbon atoms, or by fluorine, phenyl, phenoxy or NR²⁰R²¹,
in which
R²⁰ and R²¹ in each case represent hydrogen, or alkyl having up to 4 carbon atoms, which is optionally substituted by phenyl, or phenyl, and
R³ represents phenyl, which is optionally substituted by fluorine, chlorine, methyl or methoxy,
and salts thereof.

5. Process for preparing compounds of the general formula (I) 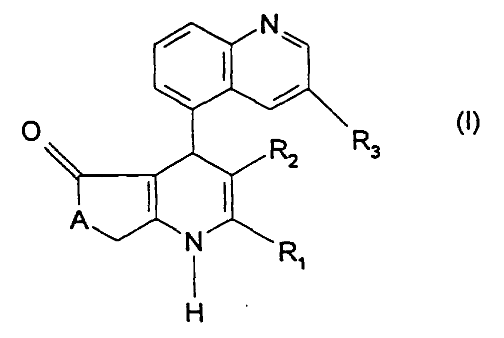 in which A, R¹, R² and R³ have the meaning specified in Claim 1, and salts thereof, characterized in that
[A] compounds of the general formula (II) 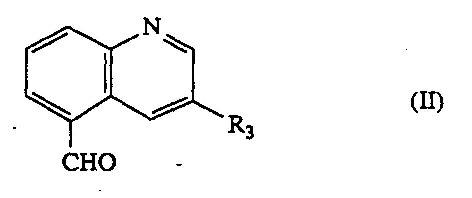 in which
R³ has the abovementioned meaning,
are initially reacted with acyl compounds of the general formula (III)
L-CO-CH₂-R² (III)
in which
R² has the abovementioned meaning
and
L has the abovementioned meaning of R¹, where, in the case of the hydroxyl and/or amino functions, these functions are optionally present in protected form,
optionally with the isolation of the ylidene compounds of the general formula (IV) 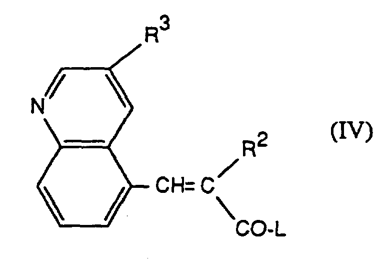 in which
R², R³ and L have the abovementioned meaning
and subsequently reacted with the compound of the formula (V) 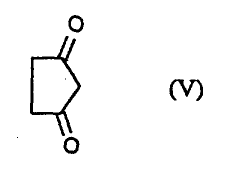 and a reactive ammonium compound, optionally with the isolation of the intermediates of the general formula (VI) 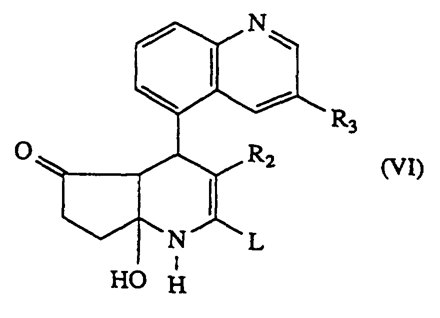 in which R², R³ and L have the abovementioned meaning, in inert solvents, and, in the case of the compounds of the general formula (VI), water is eliminated, in a last step, in the presence of an auxiliary agent, or
[B] compounds of the general formula (II) are reacted directly with the compound of the formula (V) and compounds of the general formula (VII) 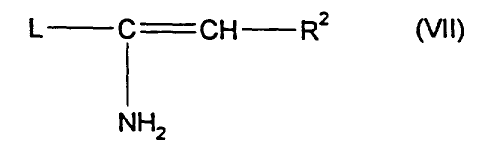 in which L and R² have the abovementioned meaning.

6. Compounds of the general formula (I) according to Claim 1 for use in the treatment of angiocardiopathies.

7. Medicaments containing at least one compound of the general formula (I) according to Claim 1.

8. Process for preparing medicaments, characterized in that at least one compound of the general formula (I) according to Claim 1, optionally together with customary auxiliary and carrier substances, is converted into a suitable form for administration.

9. Use of compounds of the general formula (I) according to Claim 1 in medicaments having a positive inotropic effect.

## Revendications

1. 4-quinolyl-dihydropyridines de formule générale (I) 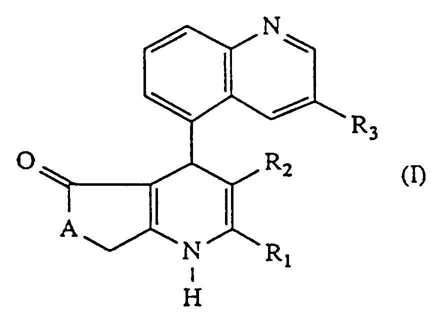 dans laquelle
A représente le groupe CH₂,
R¹ est de l'hydrogène, un groupe amino, cyano, formyle, trifluorométhyle ou un groupe alkyle linéaire ou ramifié ayant jusqu'à 8 atomes de carbone, qui est substitué, le cas échéant, par un groupe hydroxy ou par un groupe de formule -NR⁶R⁷, -O-CO-R⁸, -O-(CH₂)ₐOR^{8'} ou -O-(CH₂)_{b'}-NR⁹R¹⁰,
où
R⁶, R⁷, R⁹ et R¹⁰ sont identiques ou différents et représentent de l'hydrogène, un groupe phényle ou un groupe alkyle linéaire ou ramifié ayant jusqu'ã 6 atomes de carbone,
R⁸ et R^{8'} sont identiques ou différents et représentent un groupe alkyle linéaire ou ramifié ayant jusqu'à 6 atomes de carbone,
et
a et b sont égaux ou différents et représentent le nombre 2, 3, 4 ou 5,
R² est un groupe de formule -CO-NR¹¹R¹² ou -CO-D-R¹³,
dans laquelle
R¹¹ et R¹² sont identiques ou différents et représentent de l'hydrogène, un reste d'hydrocarbure saturé ou non saturé, à chaîne droite, à chaîne ramifiée ou cyclique ayant jusqu'à 8 atomes de carbone, qui est substitué, le cas échéant par un halogène, un radical hydroxy, cyano ou par un radical aryle, aryloxy, arylthio ayant chacun jusqu'à 6 à 10 atomes de carbone ou par un hétérocycle pentagonal à heptagonal saturé ou non saturé ayant jusqu'à 3 hétéroatomes de la série S, N ou O, les cycles pouvant, quant à eux, être substitués par un halogène, un radical cyano ou un radical alkyle, alkoxy, alkylthio, alkoxycarbonyle, halogénalkyle, halogénalkoxy ou halogénalkylthio linéaire ou ramifié ayant chacun jusqu'à 4 atomes de carbone, ou bien
un groupe aryle ayant 6 à 10 atomes de carbone ou un hétérocycle pentagonal à heptagonal saturé ou non saturé ayant jusqu'à 3 hétéroatomes de la série S, N ou O, qui sont substitués, le cas échéant jusqu'à 2 fois identiques ou différentes par un radical halogéno, cyano ou un radical alkyle, alkoxy, alkylthio, alkoxycarbonyle, halogénalkyle, halogénalkoxy ou halogénalkylthio linéaire ou ramifié ayant chacun jusqu'à 4 atomes de carbone,
ou bien
R¹¹ et R¹² forment, conjointement avec l'atome d'azote, un hétérocycle saturé ou non saturé de 3 à 8 chaînons qui est interrompu, le cas échéant, par un atome d'oxygène ou par un reste de formule S(O)_{d}, -CO- ou -NR¹⁵,
où
d est le nombre 0, 1 ou 2,
R¹⁵ est de l'hydrogène ou un groupe aryle ayant 6 à 10 atomes de carbone, qui est substitué, le cas échéant, jusqu'à 2 fois identiques ou différentes par un radical halogéno, cyano ou un radical alkyle, alkoxy, alkylthio, alkoxycarbonyle linéaire ou ramifié ayant chacun jusqu'à 8 atomes de carbone ou par un radical halogénalkyle, halogénalkoxy ou halogénalkylthio ayant chacun jusqu'à 4 atomes de carbone, ou bien
un reste hydrocarboné cyclique, linéaire ou ramifié, saturé ou non saturé ayant jusqu'à 8 atomes de carbone, qui est substitué, le cas échéant, par un radical hydroxy, halogéno ou par un radical aryle ayant 6 à 10 atomes de carbone ou par un hétérocycle pentagonal à heptagonal, saturé ou non saturé ayant jusqu'à 3 hétéroatomes de la série S, N ou O, les cycles pouvant être substitués, quant à eux, jusqu'à 2 fois identiques ou différentes par un radical halogéno, cyano ou un radical alkyle, alkoxy, alkylthio, alkoxycarbonyle, halogénalkyle, halogénalkoxy ou halogénalkylthio linéaire ou ramifié ayant chacun jusqu'à 4 atomes de carbone,
et l'hétérocycle est substitué, le cas échéant, par un radical alkoxy ou alkylthio linéaire ou ramifié ayant chacun jusqu'à 4 atomes de carbone, un halogène, un radical aryle de 6 à 10 atomes de carbone, un hétérocycle pentagonal à heptagonal, saturé ou non saturé ayant jusqu'à 3 hétéroatomes de la série S, N ou O ou par un radical alkyle linéaire ou ramifié ayant jusqu'à 4 atomes de carbone, qui peut être substitué de son côté par un radical aryle ayant 6 à 10 atomes de carbone,
D est une liaison directe ou un atome d'oxygène, R¹³ est de l'hydrogène ou un groupe aryle ayant 6 à 10 atomes de carbone ou un hétérocycle pentagonal à heptagonal, saturé ou non saturé ayant jusqu'à 3 hétéroatomes de la série S, N ou O, les cycles étant substitués, le cas échéant, jusqu'à 3 fois identiques ou différentes par un radical halogéno, cyano ou un radical alkyle, alkoxy, alkylthio, alkoxycarbonyle, halogénalkyle, halogénalkoxy ou halogénalkylthio linéaire ou ramifié ayant chacun jusqu'à 4 atomes de carbone, ou bien
un reste hydrocarboné saturé ou non saturé, cyclique, linéaire ou ramifié ayant jusqu'à 10 atomes de carbone, qui est interrompu, le cas échéant, jusqu'à 3 fois identiques ou différentes par de l'oxygène ou par un groupe -CO-, -CO-NH-, -O-CO-, -CO-O-, -NH-CO-, -SO₂-NH-, -NH-SO₂-, -S(O)ₑ- ou -NR¹⁶
où
e a la définition indiquée ci-dessus pour d et est égal à d ou en est différent,
R¹⁶ a la définition indiquée ci-dessus pour R¹⁵ et est identique à R¹⁵ ou en est différent,
ou bien le reste carboné est interrompu jusqu'à 3 fois identiques ou différentes par un groupe arylidène ayant 6 à 10 atomes de carbone ou par des restes hétérocycliques de formules 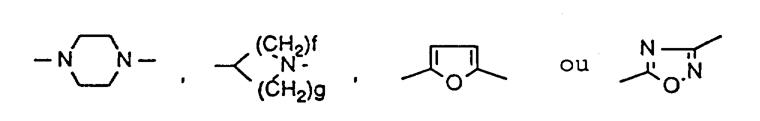 dans lesquelles
f et g sont égaux ou différents et représentent le nombre 1 ou 2,
les cycles aryliques et les hétérocycles pouvant être substitués de leur côté par un halogène, un groupe cyano ou un groupe alkyle, alkoxy, alkylthio, alkoxycarbonyle, halogénalkyle, halogénalkoxy ou halogénalkylthio linéaire ou ramifié ayant chacun jusqu'à 4 atomes de carbone,
et le reste hydrocarboné est substitué, le cas échéant, jusqu'à 3 fois identiques ou différentes par un radical cycloalkyle de 3 à 8 atomes de carbone, halogéno, nitro, cyano, hydroxy, -O-NO₂, alkylthio, alkoxy ou acyloxy linéaire ou ramifié ayant chacun jusqu'à 8 atomes de carbone ou par un radical aryle, aryloxy ou arylthio ayant chacun 6 à 10 atomes de carbone ou par un hétérocycle pentagonal à heptagonal, saturé ou non saturé ayant jusqu'à 3 hétéroatomes de la série S, N ou O, les cycles pouvant être substitués, quant à eux, jusqu'à 3 fois identiques ou différentes par un radical halogéno, un radical cyano ou un radical alkyle, alkoxy, alkylthio, alkoxycarbonyle, halogénalkyle, halogénalkoxy ou halogénalkylthio linéaire ou ramifié ayant chacun jusqu'à 4 atomes de carbone, ou bien le reste hydrocarboné peut être substitué par un groupe de formule -CO₂-R¹⁷, -CONR¹⁸R¹⁹ ou -NR²⁰R²¹,
où
R¹⁷ a la définition indiquée ci-dessus pour R¹⁵ et est identique à R¹⁵ ou en est différent et
R¹⁸, R¹⁹, R²⁰ et R²¹ ont la définition indiquée ci-dessus pour R¹¹ et R¹² et sont identiques à R¹¹ et R¹² ou sont différents
et
R³ est un groupe aryle ayant 6 à 10 atomes de carbone, qui est substitué, le cas échéant, jusqu'à 2 fois identiques ou différentes par un radical halogéno, nitro, cyano, trifluorométhyle, trifluorométhoxy, trifluorométhylthio, alkyle, alkoxy ou alkoxycarbonyle linéaire ou ramifié ayant chacun jusqu'à 8 atomes de carbone ou par un radical carboxy, ou représente un groupe thiényle ou un groupe pyridyle qui sont éventuellement substitués par un halogène
et leurs sels.

2. Composés de formule générale (I) suivant la revendication 1, dans laquelle
A représente le groupe CH₂-,
R¹ est de l'hydrogène, un groupe amino, cyano, formyle, trifluorométhyle ou un groupe alkyle linéaire ou ramifié ayant jusqu'à 6 atomes de carbone, qui est substitué, le cas échéant, par un groupe hydroxy ou par un groupe de formule -NR⁶R⁷, -O-CO-R⁸, -O-(CH₂)ₐ-OR^{8'} ou -O-(CH₂)_{b'}-NR⁹R¹⁰,
où
R⁶, R⁷, R⁹ et R¹⁰ sont identiques ou différents et représentent de l'hydrogène ou un groupe alkyle linéaire ou ramifié ayant jusqu'à 4 atomes de carbone,
R⁸ et R^{8'} sont identiques ou différents et représentent un groupe alkyle linéaire ou ramifié ayant jusqu'à 4 atomes de carbone,
et
a et b sont égaux ou différents et représentent le nombre 2, 3 ou 4,
R² est un groupe de formule -CO-NR¹¹R¹² ou -CO-D-R¹³,
dans laquelle
R¹¹ et R¹² sont identiques ou différents et représentent de l'hydrogène, un reste hydrocarboné saturé ou non saturé, linéaire, ramifié ou cyclique ayant jusqu'à 6 atomes de carbone, qui est substitué, le cas échéant, par du fluor, du chlore, un radical hydroxy, cyano ou un radical phényle, phényloxy, phénylthio ou pyridyle, les cycles pouvant être substitués, quant à eux, par du fluor, du chlore ou un radical alkyle, alkoxy, alkoxycarbonyle ayant chacun jusqu'à 2 atomes de carbone, trifluorométhyle ou trifluorométhoxy, ou bien
un groupe phényle ou un groupe pyridyle qui sont substitués, le cas échéant, par du fluor, du chlore ou par un radical alkyle, alkoxy, alkylthio, alkoxycarbonyle linéaire ou ramifié ayant chacun jusqu'à 4 atomes de carbone, trifluorométhyle ou trifluorométhoxy,
ou bien
R¹¹ et R¹² forment, conjointement avec l'atome d'azote, un hétérocycle saturé ou non saturé pentagonal à heptagonal qui est interrompu, le cas échéant, par un atome d'oxygène ou par un reste de formule S(O)_{d}, -CO- ou -NR¹⁵,
où
d représente le nombre 0, 1 ou 2,
R¹⁵ est de l'hydrogène ou un groupe phényle qui est substitué, le cas échéant,par du fluor, du chlore, un radical alkyle ou alkoxy linéaire ou ramifié ayant dans chaque cas jusqu'à 4 atomes de carbone, trifluorométhyle ou trifluorométhoxy, ou bien
un reste hydrocarboné cyclique, saturé ou non saturé, linéaire ou ramifié ayant jusqu'à 4 atomes de carbone, qui est substitué, le cas échéant, par du fluor, du chlore, un radical phényle ou un hétérocycle saturé ou non saturé, pentagonal à heptagonal ayant jusqu'à 2 hétéroatomes de la série S, N ou O, qui peuvent être être substitués, quant à eux, par du fluor, du chlore, un radical alkyle ou alkoxy linéaire ou ramifié ayant chacun jusqu'à 4 atomes de carbone, trifluorométhyle ou trifluorométhoxy, D désigne une liaison directe ou un atome d'oxygène,
R¹³ est de l'hydrogène ou un groupe phényle ou un groupe pyridyle qui sont substitués, le cas échéant, par du fluor, du chlore ou par un radical alkyle, alkoxy, alkylthio, alkoxycarbonyle linéaire ou ramifié ayant chacun jusqu'à 4 atomes de carbone, trifluorométhyle ou trifluorométhoxy, ou bien
un reste hydrocarboné saturé ou non saturé, cyclique, linéaire ou ramifié ayant jusqu'à 8 atomes de carbone, qui est interrompu, le cas échéant, jusqu'à 2 fois identiques ou différentes par un atome d'oxygène ou par un groupe -CO-, -CO-NH-, -O-CO-, -CO-O-, -NH-CO-, -SO₂-NH-, -NH-SO₂-, -S(O)ₑ- ou -NR¹⁶, où
e a la définition indiquée ci-dessus pour d et est égal à d ou en est différent,
R¹⁶ a la définition indiquée ci-dessus pour R¹⁵ et est identique à R¹⁵ ou en est différent,
ou bien le reste hydrocarboné est interrompu, le cas échéant, par un reste phénylidène ou par des restes hétérocycliques de formules 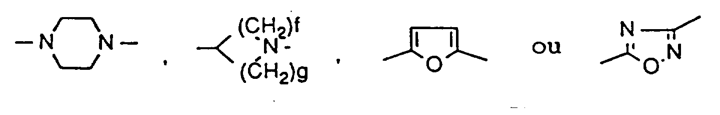 dans lesquelles
f et g sont égaux ou différents et représentent le nombre 1 ou 2,
et le reste hydrocarboné est substitué, le cas échéant, jusqu'à 2 fois identiques ou différentes par un radical cyclopropyle, cyclobutyle, cyclopentyle, cyclohexyle, fluoro, chloro, nitro, cyano, hydroxy, -O-NO₂, un radical alkylthio, alkoxy ou acyloxy linéaire ou ramifié ayant dans chaque cas jusqu'à 4 atomes de carbone ou par un radical phényle, un radical phénoxy, un radical phénylthio ou un radical pyridyle qui peuvent être substitués, quant à eux, jusqu'à 2 fois identiques ou différentes par du fluor, du chlore, un radical cyano ou par un radical alkyle, alkoxy, alkylthio, alkoxycarbonyle linéaire ou ramifié ayant chacun jusqu'à 4 atomes de carbone, trifluorométhyle ou trifluorométhoxy,
ou bien le reste hydrocarboné peut être substitué par un groupe de formule -CO₂-R¹⁷, -CONR¹⁸R¹⁹ ou -NR²⁰R²¹,
dans laquelle
R¹⁷ a la définition indiquée ci-dessus pour R¹⁵ et est égal à R¹⁵ ou en est différent et
R¹⁸, R¹⁹, R²⁰ et R²¹ ont la définition indiquée ci-dessus pour R¹¹ et R¹² et sont égaux à R¹¹ et R¹² ou en sont différents
et
R³ est un groupe phényle qui est substitué, le cas échéant, par du fluor, du chlore, un radical nitro, cyano, trifluorométhyle ou par un radical alkyle ou un radical alkoxy linéaire ou ramifié ayant chacun jusqu'à 6 atomes de carbone ou un radical hydroxy, ou bien
un groupe thiényle ou un groupe pyridyle, qui sont substitués, le cas échéant, par du fluor, du chlore ou du brome,
et leurs sels.

3. Composés de formule générale (I) suivant la revendication 1, dans laquelle
A représente le groupe CH₂-,
R¹ est de l'hydrogène, un groupe amino, cyano, formyle, trifluorométhyle ou un groupe alkyle linéaire ou ramifié ayant jusqu'à 3 atomes de carbone, qui est substitué, le cas échéant, par un radical hydroxy ou un groupe de formule -NR⁶R⁷, -O-CO-R⁸, -O-(CH₂)ₐ-OR^{8'} ou -O-(CH₂)_{b'}-NR⁹R¹⁰,
dans laquelle
R⁶, R⁷, R⁹ et R¹⁰ sont identiques ou différents et représentent de l'hydrogène, un radical méthyle ou éthyle,
R⁸ et R^{8'} sont identiques ou différents et représentent un radical méthyle ou éthyle,
et
a et b sont égaux ou différents et représentent le nombre 2 ou 3,
R² est un groupe de formule -CO-NR¹¹R¹² ou -CO-D-R¹³,
dans laquelle
R¹¹ et R¹² sont identiques ou différents et représentent de l'hydrogène, un reste hydrocarboné saturé ou non saturé, linéaire, ramifié ou cyclique ayant jusqu'à 6 atomes de carbone, qui est substitué, le cas échéant, par du fluor, du chlore, un radical phényle ou pyridyle, ou désignent un groupe phényle ou un groupe pyridyle qui sont substitués, le cas échéant, par du fluor, du chlore, un radical méthyle ou méthoxy,
ou bien
R¹¹ et R¹² forment, conjointement avec l'atome d'azote, un hétérocycle pentagonal ou hexagonal qui est interrompu, le cas échéant, par de l'oxygène ou du soufre ou le reste -NR¹⁵,
dans lequel
R¹⁵ est de l'hydrogène ou un groupe phényle qui est substitué, le cas échéant, par du fluor, du chlore, un radical méthyle, méthoxy ou trifluorométhyle, ou bien
un reste hydrocarboné cyclique, saturé ou non saturé, linéaire ou ramifié ayant jusqu'à 4 atomes de carbone, qui est substitué, le cas échéant, par un radical phényle ou pyridyle, D est une liaison directe ou un atome d'oxygène, R¹³ représente de l'hydrogène ou un groupe phényle qui est substitué, le cas échéant, par du fluor, du chlore, un radical méthyle, méthoxy ou trifluorométhyle, ou bien
un reste hydrocarboné cyclique, linéaire ou ramifié, saturé ou non saturé ayant jusqu'à 6 atomes de carbone, qui est interrompu, le cas échéant, par de l'oxygène ou du soufre ou par un groupe -CO-, -CO-NH-, -O-CO-, -CO-O-, -NH-CO-, -SO₂-NH-, -NH-SO₂- ou -NR¹⁶,
où
R¹⁶ a la définition indiquée ci-dessus pour R¹⁵ et est identique à R¹⁵ ou en est différent,
ou bien le reste hydrocarboné est interrompu, le cas échéant, par un reste phénylidène ou par des restes hétérocycliques de formules 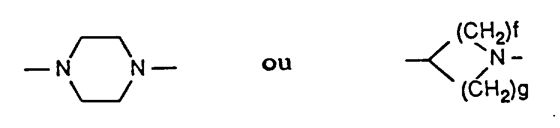 dans lesquelles
f et g sont égaux ou différents et représentent le nombre 1 ou 2,
et le reste hydrocarboné est substitué, le cas échéant, par un radical cyclopropyle, cyclobutyle, cyclopentyle, cyclohexyle, fluoro, chloro, nitro, hydroxy, un radical alkylthio ou un radical alkoxy linéaire ou ramifié ayant chacun jusqu'à 5 atomes de carbone ou par un radical phényle, un radical phénoxy, un radical phénylthio ou un radical pyridyle qui peuvent être substitués, quant à eux, par du fluor, du chlore, un radical méthyle, méthoxy, méthylthio,
ou bien
peut être substitué par un groupe de formule -CO₂-R¹⁷, -CONR¹⁸R¹⁹ ou -NR²⁰R²¹,
dans laquelle
R¹⁷ a la définition indiquée ci-dessus pour R¹⁵ et est identique à R¹⁵ ou en est différent et
R¹⁸, R¹⁹, R²⁰ et R²¹ ont la définition indiquée ci-dessus pour R¹¹ et R¹² et sont identiques à R¹¹ et R¹² ou en sont différents
et
R³ est un groupe phényle qui est substitué, le cas échéant, par du fluor, du chlore, un radical nitro, trifluorométhyle ou par un radical alkyle ou un radical alkoxy linéaire ou ramifié ayant chacun jusqu'à 4 atomes de carbone, ou représente
un groupe thiényle qui est substitué, le cas échéant, par du fluor ou du chlore,
et leurs sels.

4. Composés de formule générale (I) suivant la revendication 1, dans laquelle
A représente le groupe CH₂-,
R¹ est de l'hydrogène, un groupe amino ou méthyle, R² est un groupe de formule -CO-NR¹¹R¹² ou -CO-D-R¹³,
dans laquelle
R¹¹ et R¹² sont identiques ou différents et représentent de l'hydrogène ou un reste alkyle linéaire, ramifié ou cyclique ayant jusqu'à 6 atomes de carbone,
D est un atome d'oxygène,
R¹³ est de l'hydrogène ou un groupe alkyle linéaire ou ramifié ayant jusqu'à 6 atomes de carbone, qui est substitué, le cas échéant, par un radical cyclopropyle, cyclobutyle, cyclopentyle, cyclohexyle, un radical alkoxy ou alkylthio linéaire ou ramifié ayant chacun jusqu'à 4 atomes de carbone, du fluor, un radical phényle, phénoxy ou un radical de formule NR²⁰R²¹,
dans laquelle
R²⁰ et R²¹ représentent chacun de l'hydrogène, un radical alkyle ayant jusqu'à 4 atomes de carbone qui est substitué, le cas échéant, par un radical phényle, ou bien le radical phényle,
et
R³ est un radical phényle qui est substitué, le cas échéant, par du fluor, du chlore, un radical méthyle ou méthoxy,
et leurs sels.

5. Procédé de production de composés de formule générale (I) 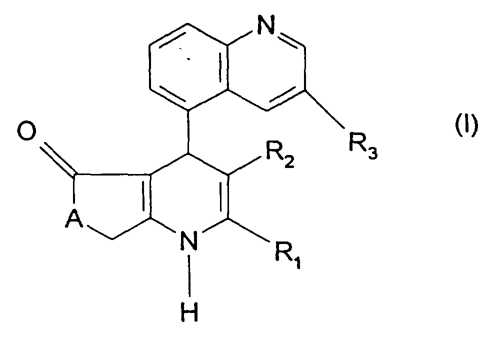 dans laquelle A, R¹, R² et R³ ont la définition indiquée dans la revendication 1,
et de leurs sels, caractérisé en ce que :
[A] on fait réagir des composés de formule générale (II) 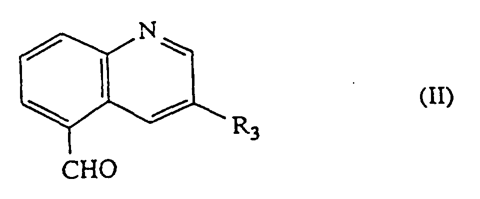 dans laquelle
R³ a la définition indiquée ci-dessus,
tout d'abord avec des composés acylés de formule générale (III)
L-CO-CH₂-R² (III)
dans laquelle
R² a la définition indiquée ci-dessus et
L a la définition indiquée ci-dessus pour R¹, et dans le cas des fonctions hydroxy et/ou amino, ces fonctions se présentent éventuellement sous la forme protégée,
le cas échéant avec isolement des composés ylidéniques de formule générale (IV) 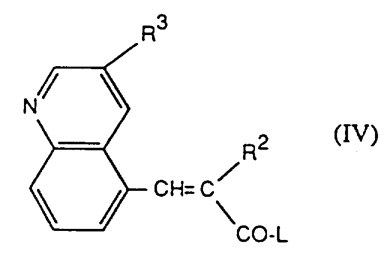 dans laquelle
R², R³ et L ont la définition indiquée ci-dessus, puis on les fait réagir avec le composé de formule (V) 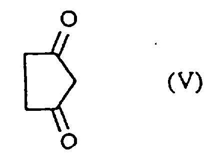 et un composé d'ammonium réactif, le cas échéant en isolant les produits intermédiaires de formule générale (VI) 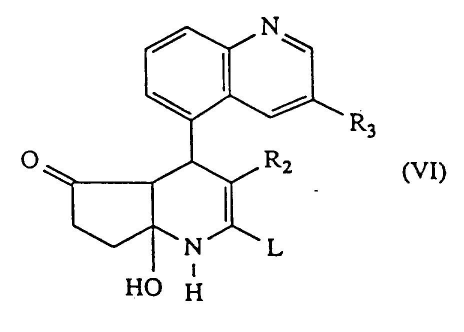 dans laquelle R², R³ et L ont la définition indiquée ci-dessus, dans des solvants inertes, et dans le cas des composés de formule générale (VI), on sépare l'eau dans une dernière étape en présence d'un agent auxiliaire, ou bien
[B] on fait réagir des composés de formule générale (II) directement avec le composé de formule (V) et des composés de formule générale (VII) 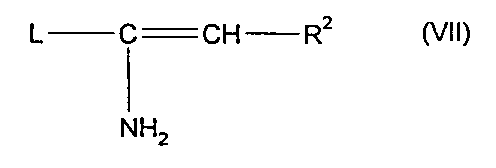 où L et R² ont la définition indiquée ci-dessus.

6. Composés de formule générale (I) suivant la revendication 1, destinés à être utilisés dans le traitement de troubles cardiaques et circulatoires.

7. Médicament contenant au moins un composé de formule générale (I) suivant la revendication 1.

8. Procédé de préparation de médicaments, caractérisé en ce qu'on fait prendre à au moins un composé de formule générale (I) suivant la revendication 1 une forme administrable appropriée, le cas échéant avec des substances auxiliaires et des supports.

9. Utilisation de composés de formule générale (I) suivant la revendication 1, dans des médicaments à action inotrope positive.
